# EUROPÄISCHE PATENTSCHRIFT

(11) **EP 2 271 304 B1**
(45) Veröffentlichungstag und Bekanntmachung des Hinweises auf die Patenterteilung: **04.09.2019**
(21) Anmeldenummer: 09725213.4
(22) Anmeldetag: 13.03.2009
(51) Int. Cl.: A61K 8/87, A61Q 5/06

(54) **HAARFESTIGER-ZUSAMMENSETZUNG**
HAIR FIXING COMPOSITION
COMPOSITION DE FIXATION DES CHEVEUX

(30) Priorität: 26.03.2008 EP 08153274
(43) Veröffentlichungstag der Anmeldung: 12.01.2011
(73) Patentinhaber: Covestro Deutschland AG, 51373 Leverkusen (DE)
(72) Erfinder: VIALA, Sophie, 50935 Köln (DE); DÖRR, Sebastian, 40597 Düsseldorf (DE); HOFACKER, Steffen, 51519 Odenthal (DE)
(74) Vertreter: Levpat
(86) Internationale Anmeldenummer: PCT/EP2009/001816
(87) Internationale Veröffentlichungsnummer: WO 2009/118105

(56) Entgegenhaltungen:
- EP-A- 0 957 119
- WO-A-02/08327
- WO-A-02/09655
- WO-A-02/09658
- WO-A-99/39688
- WO-A-2007/084596
- WO-A-2007/115697
- WO-A-2008/039466
- WO-A1-97/17386
- DE-A1- 4 241 118
- GB-A- 1 462 597

## Beschreibung

Die vorliegende Erfindung betrifft Haarfestiger-Zusammensetzungen, enthaltend spezielle Polyurethane sowie die Verwendung der genannten Polyurethane zur Herstellung von Haarfestiger-Zusammensetzungen.

Zur Gestaltung und Stabilisierung vielseitiger Frisuren werden Produkte eingesetzt, die als Haarfestiger (engl. Hair Styling) bekannt sind. Die Haarfestiger gibt es zumeist in Form von Schaumfestigern oder Haarsprays. Schaumfestiger und Haarsprays unterscheiden sich kaum in ihrer Zusammensetzung aber in ihrer Anwendung. Schaumfestiger werden im feuchten Haar als Hilfsmittel zur Modellierung der Frisur aufgetragen. Im Gegensatz dazu, sprüht man die Haarsprays auf trockene fertig gestylte Haare zur Fixierung der Frisur. Neben Haarsprays und Schaumfestiger werden auch Haarfestigergele angeboten.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh-oder Schäumvorrichtungen versprühbare Präparate vor, die aus einer alkoholischen oder wäßrigalkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden. Im Falle von Haarfestigergelen werden die oben beschriebenen Präparate mit herkömmlichen Verdickern auf eine akzeptable Viskosität eingestellt.

Bei der Formulierung von kosmetischen Produkten ist, wie bei technischen Anwendungen wie zum Beispiel bei Lacken, zu berücksichtigen, dass aufgrund der Umweltbestimmungen zur Kontrolle der Emission von flüchtigen organischen Verbindungen (volatile organic compound - Abkürzung: VOC) in die Atmosphäre der Anteil an Treibmitteln reduziert werden muss. Im Sinne der vorliegenden Patentanmeldung werden unter VOC organische, also Kohlenstoff-haltige Stoffe, die leicht verdampfen (flüchtig sind) bzw. schon als Gas bei niedrigen Temperaturen (z.B. Raumtemperatur (23°C)) vorliegen, verstanden. Erfindungsgemäß schließen die VOC insbesondere organische Stoffe mit Siedepunkten im Bereich von -90° bis 300°C ein, insbesondere flüchtige Kohlenwasserstoffe (z.B. Propan, Butan, i-Butan, Pentan, usw.), flüchtige Ether, wie Dimethylether, flüchtige Alkohole, wie Methanol, Ethanol, Isopropanol und n-Propanol und flüchtige Amine. Insbesondere der Anteil an VOC durch Amine und organische Lösemittel wie Alkohole soll in den kosmetischen Formulierungen reduziert werden, beispielsweise mittels Ersatz von Alkoholen durch Wasser.

Als filmbildende Polymere werden im Stand der Technik bevorzugt anionische oder amphotere Polymere auf Basis von Acrylaten eingesetzt. Der Einsatz von herkömmlichen Acrylaten in VOCarmen Haarfestigerformulierungen führt zu Problemen der Stabilität VOC-armer Zusammensetzungen, wie Sedimentation, Separation etc, wie dem Fachmann bekannt ist.

Außerdem zeigen die herkömmlichen filmbildenden Polymeren eine geringe Feuchtigkeits- und/oder Wasserresistenz, wenn die Haare im Kontakt mit Regen oder Schweiß stehen, bzw. bei Feuchtigkeitskontakt oder unter dem Einfluss hoher Luftfeuchte z.B. während des Badens.

Die Verwendung von Polyurethanen in Haarfestigern ist bekannt. EP 1049446 A beschreibt die Verwendung bestimmter Polyurethane in einer Hair Care Aerosolzusammensetzung, wobei das Ventil, die Öffnung sowie die anfänglichen Durchsatz der Aerosolzusammensetzung definiert sind. Ähnlich beschreibt die EP 1049443 A die Verwendung von bestimmten Polyurethanen in Hair Care Aerosolformulierungen, wobei die Formulierungen aus 0,1 bis 20 % Polykondensat aus Polyurethanen und/oder Polyharnstoff, 7,5 bis 70% organisches Lösungsmittel, 15 bis 85 % Treibmittel und 0,01 bis 20 % von mindestens einem Polyol bestehen. Bei den oben genannten Patenten wurde gefunden, dass die Sprühbarkeit des Haaraerosoles im Vergleich zum Stand der Technik verbessert ist.

Die EP 1652509 A beschreibt ein Gel, das mindestens ein Polyurethan mit einem Molekulargewicht zwischen 400 000 und 5 000 000 g/mol und mindestens einen Verdicker enthält. Um die Wasserresistenz zu verbessern, schlägt die Anmelderin den Einsatz eines Polyurethans mit einem höheren Molekulargewicht gegenüber den Polymeren des Standes der Technik vor. Die in der Erfindung beschriebenen Vorteile des Einsatzes eines Polyurethans mit einem hohen Molekulargewicht waren erwartbar. Doch ist der Einsatz eines solchen Polymers in Haarsprays und Schaumfestiger durch das hohe Molekulargewicht beschränkt. Es ist dem Fachmann nämlich bekannt, dass die Verwendung eines Filmbildners mit einem zu hohen Molekulargewicht die Sprühbarkeit der Formulierung verschlechtert.

Polyurethane für Haarfestigerzusammensetzungen sind ferner in den folgenden Patente beschrieben: EP 0751162, EP 0637600, FR 2743297, WO 9403510 und EP 0619111. Die WO 94/03510, EP 0619111 und EP 637600 beschreiben Polyurethane aus mindestens einem Säure oder Salzgruppen enthaltenden Diol, insbesondere Dimethylolpropansäure oder N-Methyl-diethanolamin. Die EP 0751162 und FR 2743297 beschreiben sequentielle Polyurethan- und/oder Polyharnstoff-Polykondensats, die aus mindestens einem Polysiloxanblock (FR 2743297) oder aus mindestens einem Polysiloxanpfropfzweige enthaltenden Polyurethan und/oder Polyharnstoffblock (EP 0751162) bestehen, wobei der Polyurethanblock aus mindestens einem Säure- oder Salzgruppen enthaltenden Diol, insbesondere Dimethylolpropansäure besteht.

EP 1457196 A beschreibt eine Hair Care Zusammensetzung für Aerosol, die aus Wasser, mindestens einem organischen Lösungsmittel, mindestens einem Polyurethan und mindestens einem Treibmittel aus Dimethylether (DME) und mindestens einem C3-4 Kohlenwasserstoff besteht. Die bevorzugten Polyurethane bestehen aus einem divalenten C2-C10 Radikal enthaltend eine Carbonsäure oder Sulfonsäuregruppe, bevorzugt Dimethylolpropansäure. Luviset PUR (INCI-Name: Polyurethane-1) und Luviset Si PUR A (INCI-Name : Polyurethane-6 = Copolymer aus Dimethylolpropansäure, Isophorondiisocyanat, Neopentylglykol, Polyesterdiol und Silikondiamin) werden eingesetzt zur Verbesserung der sensorischen Eigenschaften von Haarfestigersprays.

Ähnlich wie EP 1457196, beschreibt die EP 789550 eine Zusammensetzung aus 10 bis 60 wt. % DME, 39,9 bis 89,9% Wasser, 0,1 bis 15 wt.% Polyurethane und 0 bis 5 wt.% Alkanol mit 1 bis 4 Kohlenwasserstoffatomen. Die verwendeten Polyurethane sind ähnlich aufgebaut wie in EP 1457196 A.

Die DE 19541326 A beschreibt wasserlösliche oder wasserdispergierbare Polyurethane aus:
a) einem in Wasser löslichen oder dispergierbaren Urethanprepolymer mit endständigen Isocyanatgruppen und,
b) mindestens einem primären oder sekundären Amin, das mindestens eine ionogene bzw. ionische Gruppe aufweist, sowie die Salze davon.

Das Urethanprepolymer ist ein wasserlösliches oder wasserdispergierbares Polyurethan mit endständigen Isocyanatgruppen, welches ionogene oder ionische an die Polymerkette gebundene Gruppen aufweist. Die ionogenen oder ionische Gruppen können Carbonsäuregruppen und/oder Sulfonsäuregruppen und/oder stickstoffhaltige Gruppen, insbesondere Dimethylolpropansäure enthalten. Daraus resultiert eine vergleichsweise hohe Hydrophilie des Urethanprepolymer.

WO 2008/039466 A offenbart den Einsatz von isocyanatfunktionellen Polyurethanprepolymeren, die ionische bzw. ionogene Gruppen aufweisen, zur Herstellung von Polyurethanen, die in Körperpflegeprodukten eingesetzt werden können. WO 02/09655 A offenbart, dass bei der Herstellung von Polyurethanen Prepolymere eingesetzt werden, die durch Einsatz der Komponente (a)(iii) wasserdispergierbar gemacht werden. Als geeignete Komponenten (a)(iii) sind dabei ionische oder ionogene Gruppen angegeben. Die Schrift WO 02/09658 A offenbart Polyurethanprepolymere, die stets eine Komponente enthalten, die eine Carboxylgruppe trägt. EP 0 957 119 A offenbart, dass bei der Synthese der zur Herstellung von Polyurethanen verwendeten Polyurethanprepolymere stets ionische Reagenzien c) eingesetzt werden. Die WO 99/39688 A offenbart eine hydrophilierende Komponente C), die stets Carboxylgruppen trägt, für die Herstellung von Polyurethanen. In den Schriften WO 02/08327 A und WO 2007/084596 A werden Polyurethane offenbart, die durch Umsetzung von isocyanatfunktionellen Prepolymeren mit aminischen Kettenverlängerern, die keine Sulfonat- oder Sulfonsauregruppen tragen, aufgebaut werden. GB 1 462 597 A offenbart eine Beschichtung für Textilien. Die WO 2007/115697 A offenbart die Verwendung von anionisch hydrophilierten, wassrigen Polyurethandispersionen zur Herstellung von geschäumten Wundauflagen.Die primären oder sekundären Amine reagieren mit den endständigen Isocyanatgruppen des Urethanprepolymers und werden über eine Harnstoffgruppierung an das Polyurethan gebunden. Bei den Aminen handelt es sich besonders bevorzugt um Taurin, N-(1,1-dimethyl-2-hydroxyethyl)-3-amino-2-hydroxy-propansulfonsäure oder 4-Aminobenzolsulfonsäure. Die Anwendung der genannten Polyurethane als Hilfsmittel in der Kosmetik insbesondere als Haarfestiger ist erwähnt. Die Polyurethane sind im Vergleich zu anderen Polyurethanpolymeren des Standes der Technik bei Untersuchungen an künstlichen Modellköpfen leichter auswaschbar.

Die oben genannten Patente erwähnen jedoch wichtige Eigenschaften des Filmbildners wie die Feuchtigkeit- oder Wasserresistenz nicht. Die Polyurethane des Standes der Technik sind insbesondere durch Einsatz von 2-2-hydroxymethyl-subtituierten Carbonsäuren, bevorzugt Dimethylolpropansäure, hydrophil. Durch Neutralisierung der Carboxylgruppen an der Hauptkette mit Aminen oder Alkalien wird die Einarbeitung des Polyurethans in eine Formulierung mit einem niedrigem VOC möglich. Je mehr Carboxylatgruppen vorhanden sind, umso hydrophiler wird das Polymer und umso besser löst es sich in einem Lösungsmittelsystem, das einen hohen Anteil von Wasser enthält. Formulierungen mit einem niedrigen Gehalt an VOC sind mit den oben beschriebenen Polyurethanen zwar möglich, allerdings auf Kosten wichtiger Eigenschaften wie insbesondere den Lockenhalt bei hoher Feuchtigkeit. Die hohe Anzahl an Säuregruppen in der Polymerkette beschleunigt nämlich den Zusammenbruch der Frisur.

Des weiteren sind in den aktuell bekannten wässrigen Systemen auf Polyurethanbasis neben Lösemitteln vielfach auch flüchtige Amine enthalten, die als Neutralisationsmittel verwendet werden. Diese Amine können unter toxikologischen Gesichtspunkten kritisch für eine derartige, körpernahe Anwendung sein.

Ein weiterer Nachteil bei den Systemen nach dem Stand der Technik ist die hohe Menge des entsprechenden Filmbildners, die zur Erzielung der gewünschten Effekte zugesetzt werden muss. Dies schränkt die Freiheit bei der Erstellung der Formulierung ein und kann außerdem zu erhöhten Kosten führen.

Der vorliegenden Erfindung liegt daher die Aufgabe zugrunde, eine VOC-arme Haarfestiger-Zusammensetzung bereitzustellen, die eine sehr hohe Feuchtigkeitsresistenz bereits bei einer geringen Einsatzkonzentration der Polyurethane aufweist und zu einem sehr guten Lockenhalt führt.

Die vorliegende Erfindung stellt somit insbesondere VOC-arme Haarfestigerformulierungen bereit, die einen ausgezeichneten Lockenhalt selbst bei hoher Feuchtigkeit und bei geringen Einsatzkonzentrationen des Polyurethans aufweisen, im Vergleich mit den Haarfestigerformulierungen aus dem Stand der Technik.

Es wurde überraschend gefunden, dass sich zur Lösung der Aufgabe spezielle Polyurethane, insbesondere sie enthaltende wässrige Polyurethan-Dispersionen besonders gut eignen, die aus wasserunlöslichen, nicht wasserdispergierbaren isocyanatfunktionellen Prepolymeren hergestellt werden.

Gegenstand der vorliegenden Erfindung ist somit eine Haarfestiger Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen oder ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist. Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23°C weniger als 10 g / Liter, bevorzugter weniger als 5 g / Liter beträgt und das Prepolymer bei 23° keine sedimentationsstabile Dispersion in Wasser, insbesondere entionisiertem Wasser, ergibt. Mit anderen Worten setzt sich das Prepolymer, beim Versuch es in Wasser zu dispergieren, ab.

Bevorzugt weist das erfindungsgemäß verwendete Polyurethanprepolymere A) endständige Isocyanatgruppen auf, d.h. die Isocyanatgruppen liegen an den Kettenenden des Prepolymers. Besonders bevorzugt weisen sämtliche Kettenenden eines Prepolymers Isocyanatgruppen auf.

Weiterhin weist das erfindungsgemäß verwendete Polyurethanprepolymere A) im Wesentlichen weder ionische noch ionogene Gruppen auf, d.h. der Gehalt an ionischen und ionogenen Gruppen liegt unter 15 Milliequivalenten pro 100 g Polyurethanprepolymer A), bevorzugt unter 5 Milliequivalenten, besonders bevorzugt unter einem Milliequivalente und ganz besonders bevorzugt unter 0,1 Milliequivalenten pro 100 g Polyurethanprepolymer A).

Die aminofunktionellen Verbindungen B) umfassen wenigstens eine aminofunktionelle Verbindung B2), die Sulfonat- oder Sulfonsäuregruppe, noch bevorzugter die Natriumsulfonatgruppe, aufweist.

In einer weiteren bevorzugten Ausführungsform der Erfindung umfassen die aminofunktionellen Verbindungen B) sowohl aminofunktionelle Verbindungen B2), die Sulfonat- oder Sulfonsäuregruppe, noch bevorzugter die Natriumsulfonatgruppe, aufweist., als auch aminofunktionellen Verbindungen B1), die keine ionische oder ionogene Gruppe aufweisen.

Polyurethane im Sinne der Erfindung sind demnach polymere Verbindungen, die mindestens zwei bevorzugt mindestens drei Urethan-Gruppen-haltige Wiederholungseinheiten aufweisen:

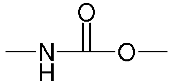

Erfindungsgemäß sind auch solche Polyurethane eingeschlossen, die herstellungsbedingt auch Harnstoff-Gruppen-haltige Wiederholungseinheiten: 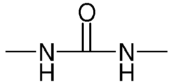 aufweisen, wie sie insbesondere bei der Umsetzung der isocyanatterminierten Prepolymere A) mit den aminofunktionellen Verbindungen B) gebildet werden.

Bei den erfindungsgemäßen Haarfestiger-Zusammensetzungen handelt es sich insbesondere um wasser-enthaltende, d.h. wässrige Zusammensetzungen, in denen das Polyurethan dispergiert, d.h. im Wesentlichen nicht gelöst vorliegt. Wasser bildet im Allgemeinen neben wahlweise vorhandenen anderen flüssigen Medien, wie beispielsweise Lösungsmitteln, den Hauptbestandteil (> 50 Gew.-%) der Dispergiermedien, bezogen auf die Gesamtmenge der flüssigen Dispergiermedien in den erfindungsgemäßen kosmetischen Zusammensetzungen, gegebenenfalls auch das alleinige flüssige Dispergiermedium.

Die erfindungsgemäßen Haarfestiger-Zusammensetzungen weisen bevorzugt einen Gehalt an flüchtigen organischen Verbindungen (VOC) von weniger als 80 Gew.-%, bevorzugter von weniger als 55 Gew.-%, noch bevorzugter von weniger als 40 Gew.-% bezogen auf die Haarfestiger-Zusammensetzung auf.

Die zur Herstellung der erfindungsgemäßen Haarfestiger-Zusammensetzungen verwendeten wässrigen Polyurethandispersionen weisen bevorzugt einen Gehalt an flüchtigen organischen Verbindungen (VOC) von weniger als 10 Gew.-%, bevorzugter von weniger als 3 Gew.-%, noch bevorzugter von weniger als 1 Gew.-% bezogen auf die auf wässrige Polyurethandispersion auf.

Die Bestimmung des Gehalts an flüchtigen organischen Verbindungen (VOC) erfolgt im Rahmen der vorliegenden Erfindung insbesondere durch gaschromatographische Analyse.

Die erfindungsgemäß verwendeten nicht wasserlöslichen und nicht-wasserdispergierbaren, isocyanatfunktionellen Polyurethanprepolymere, weisen ionische und/oder ionogene (ionenbildenden) Gruppen, wie insbesondere anionischer Gruppen, wie Carboxylat oder Sulfonat, oder kationischer Gruppen zu weniger als 15 Milliequivalente pro 100 g Polyurethanprepolymer A), bevorzugt weniger als 5 Milliequivalente, besonders bevorzugt weniger als ein Milliequivalent und ganz besonders bevorzugt weniger als 0,1 Milliequivalente pro 100 g Polyurethanprepolymer A), auf.

Im Falle saurer ionischer und/oder ionogener Gruppen beträgt die Säurezahl des Prepolymers zweckmäßig unter 30 mg KOH/g Prepolymer, bevorzugt unter 10 mg KOH/g Prepolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Die zur Herstellung der Polyurethane verwendeten Prepolymere A) sind bevorzugt erhältlich durch die Umsetzung von einem oder mehreren Polyolen, ausgewählt aus der Gruppe, die aus Polyether-Polyolen, Polycarbonatpolyolen, Polyether-Polycarbonat-Polyolen und/oder Polyesterpolyolen besteht, und Polyisocyanaten, wie weiter unten näher erläutert wird.

Die in den erfindungsgemäßen Haarfestiger-Zusammensetzungen enthaltenen Polyurethane enthalten via das Prepolymer A) demnach bevorzugt mindestens eine Sequenz, die ausgewählt wird aus der Gruppe, die besteht aus: Polyether-, Polycarbonat-, Polyether-Polycarbonat- und PolyesterSequenzen. Dies bedeutet erfindungsgemäß insbesondere, dass die Polyurethane Ethergruppen- und/oder Carbonatgruppen-haltige oder Estergruppen-Wiederholungseinheiten enthalten. Die Polyurethane können beispielsweise ausschließlich Polyether-Sequenzen oder ausschließlich Polycarbonatsequenzen oder ausschließlich Polyestersequenzen enthalten. Sie können aber auch sowohl Polyether- als auch Polycarbonat-Sequenzen aufweisen, wie sie beispielsweise bei der Herstellung von Polycarbonat-Polyolen unter Verwendung von Polyetherdiolen gebildet werden, wie unten noch ausführlich beschrieben wird. Zusätzlich können sie Polyether-Polycarbonat-Sequenzen aufweisen, welche aus der Verwendung von Polyether-Polycarbonat-Polyolen, wie weiter unten näher beschrieben, hervorgehen.

Besonders bevorzugte Polyurethane werden unter Verwendung von polymeren Polyether-Polyolen und/oder polymeren Polycarbonat-Polyolen und/oder Polyether-Polycarbonat-Polyolen oder Polyesterpolyolen erhalten, welche jeweils zahlenmittlere Molekulargewichte von bevorzugt etwa 400 bis etwa 6000 g/mol (hier und bei den folgenden Molekulargewichtangaben bestimmt durch Gelpermeationschromatographie gegenüber Polystyrol-Standard in Tetrahydofuran bei 23°C) aufweisen. Ihre Verwendung bei der Herstellung der Polyurethane bzw. Polyurethanprepolymere führt durch Umsetzung mit Polyisocyanaten zur Bildung entsprechender Polyether- und/oder Polycarbonat und/oder Polyether-Polycarbonat-Sequenzen oder Polyestersequenzen in den Polyurethanen mit entsprechendem Molgewicht dieser Sequenzen. Besonders bevorzugt sind erfindungsgemäß Polyurethane, die aus polymeren Polyether-Diolen und/oder polymeren Polycarbonat-Diolen und/oder Polyether-Polycarbonat-Polyolen oder Polyesterpolyolen mit linearem Aufbau erhalten werden.

Die erfindungsgemäßen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt beispielsweise etwa von 1000 bis 200000, bevorzugt von 5000 bis 150000. Molekulargewichte von oberhalb 200000 können unter Umständen nachteilig sein, da die Haarfestiger-Zusammensetzungen sich manchmal schlecht auswaschen lassen.

Die in den erfindungsgemäßen Haarfestiger-Zusammensetzungen enthaltenen Polyurethane werden den genannten Zusammensetzungen insbesondere als wässrige Dispersionen zugesetzt.

Bevorzugte erfindungsgemäß einzusetzende Polyurethane bzw. Polyurethan-Dispersionen sind erhältlich, in dem
A) isocyanatfunktionelle Prepolymere aus
   A1) organischen Polyisocyanaten,
   A2) polymeren Polyolen, bevorzugt mit zahlenmittleren Molekulargewichten von 400 bis 8000 g/mol (hier und bei den folgenden Molekulargewichtangaben bestimmt durch Gelpermeationschromatographie gegenüber Polystyrol-Standard in Tetrahydofuran bei 23°C), bevorzugter 400 bis 6000 g/mol und besonders bevorzugt von 600 bis 3000 g/mol, und OH-Funktionalitäten von bevorzugt 1,5 bis 6, bevorzugter 1,8 bis 3, besonders bevorzugt von 1,9 bis 2,1,
   A3) gegebenenfalls hydroxyfunktionellen Verbindungen mit Molekulargewichten von bevorzugt 62 bis 399 g/mol, und
   A4) gegebenenfalls nichtionischen Hydrophilierungsmitteln,
      hergestellt werden, und
B) deren freie NCO-Gruppen dann ganz oder teilweise
   mit einer oder mehreren aminofunktionellen Verbindungen B), wie primären und/oder sekundären Aminen und/oder Diaminen,
umsetzt. Die erfindungsgemäß verwendeten Polyurethane werden bevorzugt vor, während oder nach Schritt B) in Wasser dispergiert.

Besondere bevorzugt erfolgt im Schritt B) die Umsetzung mit einem Diamin oder mehreren Diaminen unter Kettenverlängerung. Dabei können zusätzlich monofunktionelle Amine als Kettenabbrecher zur Molekulargewichtssteuerung zugesetzt werden.

Als Komponente B) können insbesondere Amine verwendet werden, die keine ionischen bzw. ionogenen, wie anionisch hydrophilierende Gruppen aufweisen (im folgenden Komponente B1)) und es können Amine verwendet werden, die Sulfonat- oder Sulfonsäuregruppen aufweisen (im folgenden Komponente B2)).

Bevorzugt wird im Schritt B) der Umsetzung des Prepolymers eine Mischung aus Komponente B1) und Komponente B2) zur Umsetzung gebracht. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) läßt sich eine optimale Balance zwischen Hydrophilie und Kettenlänge und damit eine gute Substantivität erzielen, ohne das sich "build-up"-Effekte einstellen.

Die erfindungsgemäß verwendeten Polyurethane weisen Sulfonat- oder Sulfonsäuregruppen auf. Diese anionischen Gruppen werden in die erfindungsgemäß verwendeten Polyurethanen über die in Schritt B) umgesetzte Aminkomponente B2) eingeführt. Die erfindungsgemäß verwendeten Polyurethane weisen gegebenenfalls zusätzlich nicht-ionische Komponenten zu Hydrophilierung auf. Besonders bevorzugt sind in den erfindungsgemäß verwendeten Polyurethanen zur Hydrophilierung ausschließlich Sulfonatgruppen enthalten, welche über entsprechende Diamine als Komponente B2) in das Polyurethan eingeführt werden.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malver Inst. Limited).

Der Feststoffgehalt der Polyurethan-Dispersionen, welche zur Herstellung der Haarfestiger-Zusammensetzung der Erfindung bevorzugt verwendet wird, beträgt im allgemeinen 10 bis 70, bevorzugt 30 bis 65, besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethan-Dispersionen weniger als 5 Gew.-%, besonders bevorzugt weniger als 0,2 Gew.-%, bezogen auf die Masse der Dispersionen, an ungebundenen organischen Aminen auf. Der Gehalt in den Haarfestigerzusammensetzungen ist entsprechend noch geringer. Geeignete Polyisocyanate der Komponente A1) sind insbesondere die dem Fachmann an sich bekannten aliphatischen, aromatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4, ganz besonders bevorzugt 2.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von bevorzugt 400 bis 8000 g/mol, bevorzugter von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Der Ausdruck "polymere" Polyole bedeutet hier insbesondere, dass die genannten Polyole mindestens zwei, bevorzugter mindestens drei miteinander verbundene Wiederholungseinheiten aufweisen.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Die bevozugt verwendeten Polyesterpolyole sind die an sich bekannten Polykondensate aus Disowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Erfindungsgemäß besonders bevorzugt sind als Komponente A2) zur Herstellung der Polyurethane, Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol.

Ebenfalls können als Komponente A2) hydroxylgruppenaufweisende Polycarbonate, bevorzugt Polycarbonatdiole, mit zahlenmittleren Molekulargewichten Mₙ von bevorzugt 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können als Komponente A2) Polyetherpolyole eingesetzt werden.

Besonders geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Besonders bevorzugte Komponenten in A2) sind Polytetramethylenglykolpolyether und Polycarbonat-Polyole bzw. deren Mischungen und besonders bevorzugt sind Polytetramethylenglykolpolyether.

In bevorzugten Ausführungsformen der Erfindung handelt es sich bei Komponente A2) demnach um:
- Mischungen, enthaltend wenigstens ein Polyether-Polyol und wenigstens ein PolycarbonatPolyol,
- Mischungen, enthaltend mehr als ein Polyether-Polyol, bzw. ein Gemisch mehrerer Polyether-Polyole mit unterschiedlichen Molekulargewichten, wobei es sich insbesondere um Poly(tetramethylenglykol)polyetherpolyole (wie (HO-(CH₂-CH₂-CH₂-CH₂-O)ₓ-H) handelt,
- Mischungen, enthaltend mehr als ein Polyether-Polyol, und wenigstens ein PolycarbonatPolyol, sowie
- besonders bevorzugt Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol,
wobei die Komponente A) definitionsgemäß im Wesentlichen weder ionische noch ionogen Gruppen aufweist.

Als Komponente A3) können wahlweise Polyole, insbesondere nicht-polymere Polyole, des bevorzugt genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäurebis(ß-hydroxyethyl)-ester.

Ferner können als Komponente A3) auch monofunktionelle isocyanatreaktive Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethans nicht verwendet.

Als Komponente A4) werden zur Herstellung der erfindungsgemäß verwendeten Polyurethane gegebenenfalls ein oder mehrere insbesondere isocyanatreaktive nichtionische Hydrophilierungsmittel verwendet. Die als Komponente A4) verwendeten Hydrophilierungsmittel sind insbesondere von den Komponenten A2) und A3) verschieden.

Geeignete nichtionisch hydrophilierende Verbindungen als Komponente A4) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion ein-Die Komponente B) wird bevorzugt aus primären oder sekundären Amin und/oder Diaminen ausgewählt. Sie umfasst insbesondere Diamine.

Als Komponente B) können insbesondere Amine verwendet werden, die keine ionischen bzw. ionogenen, wie anionisch hydrophilierenden Gruppen aufweisen (im folgenden Komponente B1)), und es können Amine verwendet werden, die Sulfonat- oder Sulfonsäuregruppen aufweisen (im folgenden Komponente B2)). Bevorzugt wird im Schritt B) der Umsetzung des Prepolymers eine Mischung aus der Komponente B1) und der Komponente B2) zur Umsetzung gebracht.

Beispielsweise können als Komponente B1) organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, Hydrazinhydrat, und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden als Komponente B1) 1,2-Ethylendiamin, Bis(4-aminocyclohexyl)methan, 1,4-Diaminobutan, Isophorondiamin, Ethanolamin, Diethanolamin und Diethylentriamin eingesetzt.

Besonders bevorzugt umfasst die Komponente B) mindestens eine Komponente B2). Geeignete anionisch hydrophilierende Verbindungen als Komponente B2) enthalten bevorzugt eine Sulfonsäure- oder Sulfonatgruppe, besonders bevorzugt eine Natriumsulfonatgruppe. Geeignete anionisch hydrophilierende Verbindungen als Komponente B2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure, 1,2- oder 1,3-Propylendiamin-ß-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure.

Besonders bevorzugt enthaltend die erfindungsgemäß verwendeten Polyurethane mindestens eine Sulfonatgruppe.

Zur Hydrophilierung können auch Mischungen aus anionischen Hydrophilierungsmitteln B2) und nichtionischen Hydrophilierungsmitteln A4) verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und/oder B1)
0,1 bis 25 Gew.-% Summe der Komponenten A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmitteln B2) verwendet werden.

In einer besonders bevorzugten Ausführungsform zur Herstellung der speziellen Polyurethan- Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) besonders bevorzugt 0,2 bis 4 Gew.-% an anionischen bzw. potentiell anionischen Hydrophilierungsmitteln B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und/oder B1)
0,1 bis 13,5 Gew.-% Summe der Komponenten A4) und/oder B2), wobei bezogen auf die Gesamtmengen der Komponenten A1) bis A4) und B1) bis B2) besonders bevorzugt 0,5 bis 3,0 Gew.-% an anionischen bzw. potentiell anionischen Hydrophilierungsmitteln aus B2) verwendet werden.

Die Herstellung der Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt bevorzugt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach das Aceton-Verfahren angewandt.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt.

Die Verwendung von organischen Aminen ist nicht bevorzugt.

Als Neutralisationsmittel werden bevorzugt anorganische Basen, wie wässrige Ammoniaklösung oder Natrium- bzw. Kaliumhydroxid einsetzbar.

Bevorzugt sind Natriumhydroxid und Kaliumhydroxid,
Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten B) zur Kettenverlängerung sind insbesondere organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt im allgemeinen zwischen 40 und 150 %, bevorzugt zwischen 50 und 110%, besonders bevorzugt zwischen 60 und 100 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 40 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt typischerweise weniger als 10 Gew.-%, bevorzugt weniger als 3 Gew.-%, bezogen auf die gesamte Dispersion.

Der pH-Wert der erfindungsgemäß verwendeten wässrigen Polyurethan-Dispersionen beträgt typischerweise weniger als 8,0, bevorzugt weniger als 7,5 und liegt besonders bevorzugt zwischen 5,5 und 7,5.

Vorteilhaft können die haarfestigenden Zusammensetzungen im Sinne der vorliegenden Erfindung in der Form eines Sprays, eines Schaums, eines Gels, einer Emulsion, einer Lösung oder einer Creme vorliegen, wie als Schaumfestiger, Flüssigfestiger, Haarspray, Stylinggel, Stylingcreme, Schaumaerosol etc..

Die erfindungsgemäße Haarfestiger-Zusammensetzung enthält bevorzugt 0,1 bis 20 Gewichts-% des oben beschriebenen Polyurethans und insbesondere 0,5 bis 10 Gewichts-% jeweils bezogen auf das Gesamtgewicht der Zusammensetzung.

Die erfindungsgemäße Zusammensetzung kann neben dem oben beschriebenen Polyurethan weitere geeignete Filmbildner enthalten, welche insbesondere auch zur Festigung und zum Styling der Haare beitragen können.

Die Konzentration eines oder mehrerer weiterer Filmbildner kann von 0 bis 20 Gewichts-% und insbesondere 0 bis 10 Gewichts-% jeweils bezogen auf das Gesamtgewicht der Zusammensetzung betragen.

Vorteilhaft werden der oder die Filmbildner ausgewählt aus der Gruppe der von den erfindungsgemäß verwendeten Polyurethane verschiedenen wasserlöslichen oder wasserdispergierbaren Polyurethane, der Polyharnstoffe, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Zusammensetzungen alleine oder im Gemisch, vorzugsweise auch mit anionischen und/oder amphoteren und/oder zwitterionischen Polymeren enthalten sein können, sind ausgewählt aus:
- Polyalkyloxazoline,
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester,
- Acrylsäureester Copolymerisate wie z. B. die Copolymerisate aus Alkyl-Acrylat und Alkyl-Methacrylat, Copolymerisate aus Alkyl-Acrylat und Urethanen,
- Copolymerisate aus Acrylnitril und nichtionischem monomer ausgewählt aus Butadien und (Meth)Acrylat,
- Styrol Homo- und Copolymerisate. Hierzu gehören beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin,
- Polyamide,
- Vinyllactame Homo- oder Copolymere, wie Vinylpyrrolidon-Homo- oder Copolymerisate; hierzu gehören beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat,
- Polysiloxane,
- Homopolymere des N-Vinylformamids z. B. PVF von National Starch.

Besondere bevorzugte nichtionische Polymere sind Acrylsäureester Copolymerisate, Homopolymere des Vinylpyrrolidons und Copolymere, Polyvinylcaprolactam.

Ganz besondere bevorzugte nichtionische Polymere sind Homopolymere des Vinylpyrrolidons z. B. Luviskol® K von der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure Homo- oder Copolymer oder die Salze davon. Hierzu gehören beispielweise die Copolymerisate aus Acrylsäure und Acrylamide und/oder deren Natriumsalze, Copolymerisate aus Acrylsäure und/oder Methacrylsäure und einem ungesättigten Monomer ausgewählt aus Ethylene, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone® LM der Firma ISP, Copolymerisate aus Methacrylsäure, Ethylacrylate und Tert-butyl Acrylate z.B. Luvimer® 100 P der Firma BASF.
- Crotonsäurederivat-Homo- oder Copolymer oder die Salze davon. Hierzu gehören beispielweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere,
- ungesättigte C4-C8 Carbonsäurederivate oder Carbonsäureanhydrid Copolymer ausgewählt aus Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogeriderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylester, Methallylester und ggfs. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser loesliche oder dispergierbare anionische Polyurethane, z. B. Luviset ®PUR von BASF, die von den erfindungsgemäßen Polyurethanen verschieden sind,
wobei diese Aufstellung selbstverständlich nicht limitierend sein soll.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidesulfonsäure.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester und Salze von Polystyrolsulfonsäure.

Ganz besondere vorteilhafte anionische Polymere sind Acrylat-Copolymere, z.B. Luvimer von BASF, Ethylacrylat/N-tert.-Butylacrylamid/Acrylsäure-Copolymere ULTRAHOLD® STRONG der Firma BASF, VA/Crotonat/Vinylneodecanoat-Copolymer z. B. Resyn 28-2930 von National Starch, Copolymerisate wie. Z. Copolymerisate aus Methylvinylether und Maleinsäureanhydrid teilverestert z.B. GANTREZ® der Firma ISP und Natrium-Polystyrol-Sulfonate z. B. Flexan 130 von National Starch.

Vorteilhafte amphotere Polymere können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält, worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylen-*α*/*β*-dicarbonsäureeinheit, bei der die Carbonsäuregruppen mit einem Polyamin umgesetzt wurden, das eine oder mehrere primäre oder sekundäre Aminogruppen enthält.

Besondere vorteilhafte amphotore Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, *α*-Chloracrylsäure, und einem basischen Monomer gebildet werden, das von einer Vinylverbindung abgeleitet ist, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent Nr. 3 836 537 beschrieben worden.
- Polymere mit Einheiten, die abgeleitet sind von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.

Erfindungsgemäß besonders bevorzugte N-substituierte Acrylamide oder Methacrylamide sind Verbindungen, deren Alkylgruppen 2 bis 12 Kohlenstoffatome enthalten, besonders N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.

Die sauren Comonomere sind insbesondere unter Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.

Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel abgeleitet sind:

   -[CO-R-CO-Z]-

   worin R eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bissekundäres Amin entsteht, und Z eine Gruppe bedeutet, die von einem bis-primären, mono- oder bis-sekundären Polyalkylenpolyamin abgeleitet ist, und vorzugsweise: a) in Mengenanteilen von 60 bis 100 Mol-% die Gruppen -NH-[(CH₂)ₓ-NH-]ₚ- mit x = 2 und p = 2 oder 3 oder x = 3 und p = 2, wobei diese Gruppe, von Diethylentriamin, Triethylentetramin oder Dipropylentriamin abgeleitet ist; b) in Mengenanteilen von 0 bis 40 Mol-% die Gruppe - NH-[(CH₂)ₓ-NH-]ₚ-, worin x = 2 und p = 1, die von Ethylendiamin abgeleitet ist, oder die Gruppe, die von Piperazin stammt: 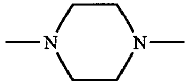 c) in Mengenanteilen von 0 bis 20 Mol-%, die Gruppe -H-(CH₂)₆-NH-, die von Hexamethylendiamin abgeleitet ist, wobei diese Polyaminoamide durch Addition eines bifunktionellen Vernetzungsmittels, das unter den Epihalohydrinen, Diepoxiden, Dianhydriden und bis-ungesättigten Derivaten ausgewählt ist, in einer Menge von 0,025 bis 0,35 mol Vernetzungsmittel pro Aminogruppe des Polyaminoamids vernetzt und mit Acrylsäure, Chloressigsäure oder einem Alkansulton oder deren Salzen acyliert sind.
   Die gesättigten Carbonsäuren sind vorzugsweise unter den Säuren mit 6 bis 10 Kohlenstoffatomen ausgewählt, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure.
   Die bei der Acylierung verwendeten Alkansultone sind vorzugsweise Propansulton oder Butansulton, die Salze der Acylierungsmittel sind vorzugsweise die Natriumsalze oder Kaliumsalze.
- Polymeren mit zwitterionischen Einheiten der folgenden Formel: 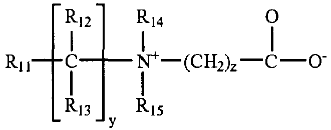 worin R₁₁ eine polymerisierbare ungesättigte Gruppe bedeutet, wie Acrylat, Methacrylat, Acrylamid oder Methacrylamid, y und z ganze Zahlen von 1 bis 3 bedeuten, R₁₂ und R₁₃ ein Wasserstoffatom, Methyl, Ethyl oder Propyl bedeuten, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt.
   Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 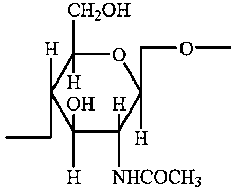 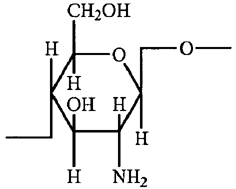 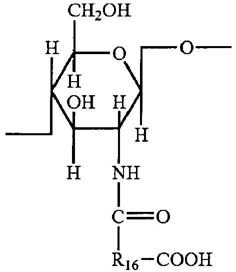 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 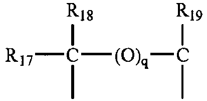 worin bedeuten: falls q = 0, die Gruppen R₁₇, R₁₈ und R₁₉, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen ist und/oder gegebenenfalls eine oder mehrere der Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoffatom, sowie die Salze, die diese Verbindungen mit Basen oder Säuren bilden.
- Polymere, die der folgenden allgemeinen Formel entsprechen und die beispielsweise in dem französischen Patent 1 400 366 beschrieben sind: 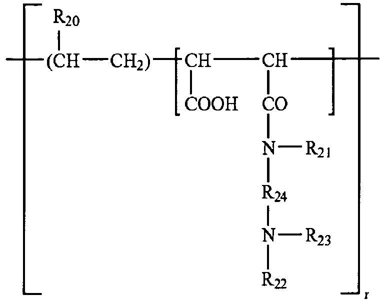 worin R₂₀ ein Wasserstoffatom, CH₃O, CH₃CH₂O oder Phenyl bedeutet, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe bedeutet, wie Methyl oder Ethyl, R₂₃ eine niedere C₁₋₆-Alkylgruppe ist, wie Methyl oder Ethyl oder eine Gruppe der Formel: -R₂₄-N(R₂₂)₂, wobei R₂₄ eine Gruppe -CH₂-CH₂, -CH₂-CH₂-CH₂- oder -CH₂-CH(CH₃)- bedeutet und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Amphotere Polymere vom Typ -D-X-D-X, die ausgewählt sind unter:
   a) Polymere, die durch Einwirkung von Chloressigsäure oder Natriumchloracetat auf Verbindungen mit mindestens einer Einheit der folgenden Formel gebildet werden: D-X-D-X,
      worin D die Gruppe bedeutet 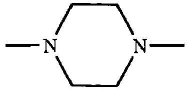 und X die Symbole E oder E' bedeutet, wobei E oder E', die gleich oder verschieden sind, eine zweiwertige Gruppe bedeuten, bei der es sich um eine geradkettige oder verzweigte Alkylengruppe mit bis zu 7 Kohlenstoffatomen in der Hauptkette handelt, die unsubstituiert vorliegt oder mit Hydroxygruppen substituiert ist und ein oder mehrere Sauerstoffatome, Stickstoffatome oder Schwefelatome und 1 bis 3 aromatische und/oder heterocyclische Ringe enthalten kann; wobei die Sauerstoffatome, Stickstoffatome und Schwefelatome in Form der folgenden Gruppen vorliegen: Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan.
   b) Polymere der Formel D-X-D-X, worin D die Gruppe bedeutet 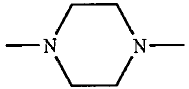 und X das Symbol E oder E' und mindestens einmal E' bedeutet; wobei E die oben angegebenen Bedeutungen aufweist und E' eine zweiwertige Gruppe ist, bei der es sich um eine geradkettige oder verzweigte Alkylengruppe mit bis zu 7 Kohlenstoffatomen in der Hauptkette handelt, die unsubstituiert vorliegt oder mit einer oder mehreren Hydroxygruppen substituiert ist und ein oder mehrere Stickstoffatome enthält, wobei das Stickstoffatom mit einer Alkylgruppe substituiert ist, die gegebenenfalls durch ein Sauerstoffatom unterbrochen ist und zwingend eine oder mehrere Carboxyfunktionen oder eine oder mehrere Hydroxyfunktionen enthält und durch Umsetzung mit Chloressigsäure oder Natriumchloracetat betainisiert ist.
      - Alkyl(C₁₋₅)vinyletherlMaleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol modifiziert sind. Diese Polymere können auch weitere Comonomere enthalten, wie Vinylcaprolactam.

Ganz besondere vorteilhafte amphotere Polymere sind z.B. die Copolymere Octylacrylamid/Acrylate/Butylamino-Ethylmethacrylat-Copolymer, die unter den Bezeichnungen AMPHOMER®, AMPHOMER® LV 71 oder BALANCE® 47 der Firma NATIONAL STARCH im Handel sind, und Methylmethacrylat/Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere die Säuregruppen enthalten können folgende Basen eingesetzt werden: Hydroxide, deren Kation ein Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS).

Die Neutralisation kann je nach Anwendungszweck teilweise oder vollständig erfolgen.

Gegebenfalls einsetzbar aber jedoch weniger bevorzugt sind kationische Polymere, wie beispielweise Polymere, die primäre, sekundäre tertiäre und/oder quaternäre Aminogruppen enthalten, die Teil der Polymerkette oder direkt an die Polymerkette gebunden sind.

Das kosmetische akzeptable Medium enthält insbesondere Wasser und gegebenenfalls ein kosmetisch geeignetes Lösungsmittel. Die bevorzugten Lösungsmittel sind aliphatische Alkohole mit C2-4 Kohlenstoffatomen wie Ethanol, Isopropanol, t-Butanol, n-Butanol; Polyol wie Propylenglycol, Glycerin, Ethylenglycol und Polyolethern; Aceton; unverzweigte oder verzweigte Kohlenwasserstoffe wie Pentan, Hexan, Isopentan und zyklische Kohlenwasserstoffe wie Cyclopentan und Cyclohexan; und deren Gemische.

Ganz besonderes bevorzugtes Lösungsmittel ist Ethanol.

Der Gehalt an derartigen Lösungsmitteln liegt aber entsprechend der Tatsache, dass es sich erfindungsgemäß bevorzugt um VOC-arme Haarfestiger-Zusammensetzungen handelt, bevorzugt bei weniger als 80 Gew.-%, noch bevorzugter bei weniger als 55 Gew.-%, noch bevorzugter bei weniger als 40 Gew.-%.

Der Wasseranteil kann insbesondere im Bereich beispielsweise von 20 bis 94 Gew.-%, bevorzugt von 30 bis 80 Gew.-%, noch bevorzugter von mehr als 45 bis 70 Gew.-%, bezogen auf das Gesamtgewicht der Zusammensetzung, liegen. Das Medium ist vorteilhaft ein wässrig-alkoholisches Gemisch. Der Mengenanteil des Alkohols im Gemisch liegt im Bereich von 0 bis 90 Gew.-%, vorzugsweise 0 bis 70 Gew.-% bevorzugter 0 bis 55 Gew.-%, noch bevorzugter bei 0 bis 40 Gew.-%, bezogen auf das Gesamtgewicht der Zusammensetzung.

Die erfindungsgemäßen Haarfestiger können des weiteren vorteilhaft Verdicker enthalten. Vorteilhafte Verdicker sind:
- Vernetzte oder nichtvernetzte Acrylsäure oder Methacrylsäure Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol® und Kelza® von der Firma CP Kelco angebotenen Produkte oder die Produkte der Firma RHODIA mit der Bezeichnung Rhodopol und Guargummi, wie die unter der Bezeichnung Jaguar® HP105 von der Firma RHODIA erhältlichen Produkte.

Ganz besondere vorteilhafte Verdicker sind vernetzte Homopolymere von Methacrylsäure oder Acrylsäure, die von der Firma Lubrizol unter den Bezeichnungen Carbopol® 940, Carbopol® 941, Carbopol® 980, Carbopol® 981, Carbopol® ETD 2001, Carbopol® EDT 2050, Carbopol® 2984, Carbopol® 5984 und Carbopol® Ultrez 10, von der Firma 3V unter den Bezeichnungen Synthalen® K, Synthalen® L und Synthalen® MS und von der Firma PROTEX unter den Bezeichnungen Modarez® V 1250 PX, Modarez® V2000 PX, Viscaron® A1600 PE und Viscaron® A700 PE im Handel erhältlich sind.

Ganz besondere vorteilhafte Verdicker sind vernetzte Copolymere von Acrylsäure oder Methacrylsäure und einem C₁₀₋₃₀-Alkylacrylat oder C₁₀₋₃₀-Alkylmethacrylat und Copolymere von Acrylsäure oder Methacrylasäure und Vinylpyrrolidon. Solche Copolymere sind beispielsweise von der Firma Lubrizol unter den Bezeichnungen Carbopol® 1342, Carbopol® 1382, Pemulen® TR1 oder Pemulen® TR2 und von der Firma ISP unter den Bezeichnungen Ultrathix P-100 (INCI : Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex® AVC (INCI: Ammonium Acryloyldimethyltaurat/VP Copolymer) erhältlich

Falls die Verdicker verwendet werden, sind sie im Allgemeinen in einer Konzentration von 0 % bis 2 Gew.-% vorzugsweise 0 % bis 1 Gew.-% bezogen auf das Gesamtgewicht der Zusammensetzung vorhanden.

Die erfindungsgemäßen Haarfestiger können des weiteren ein Treibgas enthalten. Dabei ist es vorteilhaft, das Treibgas in einer Menge von 0 bis 40 Gewichts-% und besonders bevorzugt in einer Konzentration von 0 bis 20 Gewichts-% bezogen auf das Gesamtgewicht der Formulierung einzusetzen.

Die erfindungsgemäßen bevorzugten Treibgase sind Kohlenwasserstoffe wie Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW) wie z.B. 1,2-difluoroethan (Treibmittel 152 A).

In den erfindungsgemäßen Haarfestigerformulierungen können des weiteren haarpflegende Wirkstoffe verwendet werden. Als Pflegestoffe können bevorzugt cyclische Polydimethylsiloxane (Cyclomethicone) in Konzentrationen von z. B. 0-1,0 Gew.-% der Gesamtformulierung oder Silikontenside (Polyethermodifizierte Siloxane) vom Typ Dimethicone Copolyol oder Simethicon z. B. in Konzentrationen 0-1,0 Gew.-% des Gesamtgewichts der Zusammensetzung bevorzugt zum Einsatz kommen. Cyclomethicone werden u. a. unter der Handelsbezeichnungen Abil® K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil® DM 6031 von der Firma Wacker angeboten.

Optional können herkömmliche Additive ebenfalls in den Haarfestiger- Zusammensetzung enthalten sein, beispielsweise um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.

Diese Additive sind im Allgemeinen in einer Konzentration von etwa 0,001 % bis 15 Gew.-% vorzugsweise 0,01 % bis 10 Gew.-% bezogen auf das Gesamtgewicht der Haarfestiger-Zusammensetzung vorhanden.

Die erfindungsgemäßen Haarfestiger-Zusammensetzungen können vorteilhafter Weise in einer Pumpspray- oder Aerosolverpackung vorliegen. Die erfindungsgemäßen Haarfestiger-Zusammensetzungen können vorteilhaft mit einem Treibgas aufgeschäumt werden. Dementsprechend sind Pumpspray-, Aerosolverpackungs und Schaumspender auf Pumpspray- oder Aerosolverpackungsbasis, welche die erfindungsgemäßen Haarfestiger Zusammensetzung enthalten, ebenfalls Bestandteil der Erfindung.

Eine bevorzugte Ausführungsform der erfindungsgemäßen Haarfestiger-Zusammensetzungen liegt in der Form eines Sprays vor, welches zusätzlich einen oder mehrere der folgenden Bestandteile enthält: kosmetisch geeignete Lösungsmittel, wie aliphatische Alkohole mit 2-4 Kohlenstoffatomen, bevorzugt Ethanol, Polyole, Aceton, unverzweigte oder verzweigte Kohlenwasserstoffe, zyklische Kohlenwasserstoffe und deren Gemische, sowie Treibgase wie Kohlenwasserstoffe, Druckluft, Kohlendioxid, Stickstoff, Stickstoffdioxid, Dimethylether, Fluorkohlenwasserstoffe und Fluorchlorkohlenwasserstoffe, bevorzugt Dimethylether und/oder ein Propan/Butan-Gemisch.

Die vorliegende Erfindung wird an Hand der folgenden Beispiele erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

Die vorliegende Erfindung betrifft auch die Verwendung einer kosmetischen Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist, zur Gestaltung und/oder Festigung der Haare.

Die vorliegende Erfindung betrifft auch die Verwendung von Polyurethanen, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist, zur Herstellung von kosmetischen Zusammensetzungen zur Gestaltung oder Festigung der Haare.

Die vorliegende Erfindung betrifft auch ein Verfahren zur Gestaltung oder Festigung der Haare, welches das Auftragen einer kosmetischen Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist, umfasst.

### Beispiele:

Sofern nicht abweichend gekennzeichnet, beziehen sich alle Prozentangaben auf das Gewicht.

Sofern nicht abweichend vermerkt, beziehen sich alle analytischen Messungen auf Messungen bei Temperaturen von 23°C.

Die Feststoff- bzw. Festkörpergehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt. Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen;

| | |
|---|---|
| Diaminosulfonat: | NH₂-CH₂CH₂-NH-CH₂CH₂-SO₃Na (45 %ig in Wasser) |
| Desmophen® 2020/C2200: | Polycarbonatpolyol, OH-Zahl 56 mg KOH/g, zahlenmittleres Molekulargewicht 2000 g/mol (Bayer MaterialScience AG, Leverkusen, DE) |
| PolyTHF® 2000: | Polytetramethylenglykolpolyol, OH-Zahl 56 mg KOH/g, zahlenmittleres Molekulargewicht 2000 g/mol (BASF AG, Ludwigshafen, DE) |
| PolyTHF® 1000: | Polytetramethylenglykolpolyol, OH-Zahl 112 mg KOH/g, zahlenmittleres Molekulargewicht 1000 g/mol (BASF AG, Ludwigshafen, DE) |
| Polyether LB 25: | monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE) |

### Beispiel 1: Polyurethan-Dispersion 1

987,0 g PolyTHF® 2000 (Komponente A2)), 375,4 g PolyTHF® 1000 (Komponente A2)), 761,3 g Desmophen® C2200 (Komponente A2)) und 44,3 g Polyether LB 25 (Komponente A4)) wurden in einer Standard-Rührapparatur auf 70°C aufgeheizt. Anschließend wurde ein Gemisch aus 237,0 g Hexamethylendiisocyanat (Komponente A1)) und 313,2 g Isophorondiisocyanat (Komponente A1)) zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin (Komponente B1)), 116,5 g Isophorondiamin (Komponente B1)), 61,7 g Diaminosulfonat (Komponente B2)) und 1030 g Wasser zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgende Eigenschaften:

| | |
|---|---|
| Feststoffgehalt: | 61% |
| Partikelgröße (LKS): | 312 nm |
| Viskosität (Viskosimeter, 23°C): | 241 mPas |
| pH (23°C): | 6,02 |
| pH (23°C): | 7,15 |

### Beispiel 2: Polyurethan-Dispersion 2

450 g PolyTHF® 1000 (Komponente A2)) und 2100 g PolyTHF® 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin (Komponente B1)), 143,2 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 56% |
| Partikelgröße (LKS): | 276 nm |
| Viskosität: | 1000 mPas |

### Beispiel 3: Polyurethan-Dispersion 3

1649,0 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 291,7 g Hexamethylendiisocyanat (Komponente A1)) zugegeben und solange bei 100-115°C gerührt bis der theoretische NCO-Wert unterschritten war. Das fertige Prepolymer wurde mit 3450 g Aceton bei 50°C gelöst und anschließend eine Lösung aus 16,8 g Ethylendiamin (Komponente B1)), 109,7 g Diaminosulfonat (Komponente B2)) und 425 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42% |
| Partikelgröße (LKS): | 168 nm |
| Viskosität: | 425 mPas |
| pH-Wert: | 7,07 |

### Beispiel 4: Polyurethan-Dispersion 4

340 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 60,1 g Hexamethylendiisocyanat (Komponente A1)) zugegeben und solange bei 105°C gerührt bis der theoretische NCO-Wert unterschritten war. Das fertige Prepolymer wurde mit 711 g Aceton bei 50°C gelöst und anschließend eine Lösung aus 2,1 g Ethylendiamin (Komponente B1)), 32,4 g Diaminosulfonat (Komponente B2)) und 104,3 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 40 % |
| Partikelgröße (LKS): | 198 nm |
| Viskosität: | 700 mPas |
| pH-Wert: | 6,31 |

### Beispiel 5: Polyurethan-Dispersion 5

450 g PolyTHF® 1000 (Komponente A2)) und 2100 g PolyTHF® 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 351 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42% |
| Viskosität: | 1370 mPas |

### Anwendungstechnische Vergleichversuche:

Für die sogenannten "Curl Retention"-Versuche werden kommerziell europäische Mischhaare der Firma Kerling (Nutzlänge: 19 cm, Kerling Farbnummer 6/0) eingesetzt. Die Haare werden vor der Anwendung einer standardisierten Waschprozedur unterzogen. Die 15 min im Wasser eingeweichten Haare werden mit 15 Gew.-% Natriumdodecylsulfatlösung zwei Minuten shampooniert, gründlich mit warmen Wasser ausgespült, kalt trocken geföhnt und bei 22°C und 55% relativer Feuchtigkeit konditioniert. 0,5 cm breite Haarsträhnen werden feucht auf Spiralwickler gewickelt, 35 min heiß trocken geföhnt, dann mit Polymerdispersion besprüht und über Nacht konditioniert.

Für die Applikation der Polymerdispersion wird ein Sprühkopf der Firma Seaquist Perfect Dispensing GmbH verwendet: Pumpzerstäuber Type PZ 1 / 150 HV (24/410). Aus 30 cm Entfernung werden die erfindungsgemäßen Zusammensetzungen durch zügige Betätigung des Sprühkopfes auf das Haarmaterial appliziert.

Der "Curl Retention"-Versuch wird in einer speziellen klimatisierten Kammer mit einer relativen Feuchtigkeit > 98 % durchgeführt. Die Temperatur der Kammer beträgt 30°C. Die vorbereiteten Strähnen werden gleichzeitig in der Kammer gehängt. Die Länge der Locken wird zu bestimmten Zeiten an einer Skala abgelesen. Jeder Versuch wird an drei Strähnen durchgeführt.

| Versuch | A | B |
|---|---|---|
| Ethanol (Gew.%) | 50 | 50 |
| Polyurethan (Gew.% Feststoff)¹⁾ | 4 | 2 |
| Wasser (Gew.%) | ad 100 | Ad 100 |
| Sprühstöße | 12 | 4 |

| | | |
|---|---|---|
| Angegeben sind Gewichtsteile. ¹⁾: Bezogen auf Feststoff in der wässrigen Polyurethandispersion. | | |

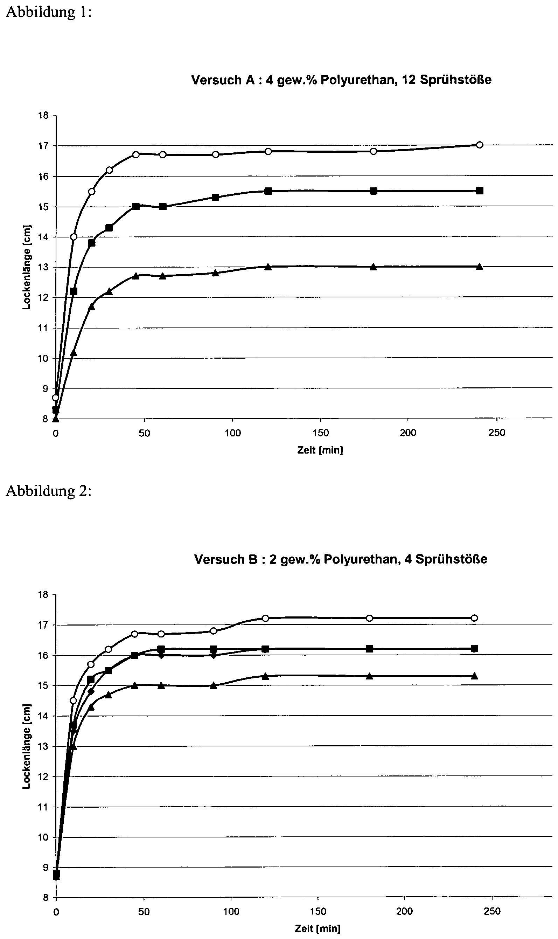

Abbildung 1 zeigt die in Versuch A erzielten Ergebnisse.

Abbildung 2 zeigt die in Versuch B erzielten Ergebnisse.

In den Abbildungen bedeutet:
O Blindversuche, ohne Polyurethan

### Polyurethane des Stands der Technik, enthaltend Dimethylpropansäure:

(Polyurethane, enthaltend Dimethylolpropansäure werden üblicherweise hergestellt über Einbau der Dimethylolpropansäure in ein Prepolymer, welches wasserlöslich oder wasserdispergierbar ist, vgl WO 94/03510, EP 0619111 und EP 637600).
▪ Polyurethan des Standes der Technik, Luviset® PUR der Firma BASF, INCI-Name: Polyurethane-1, Polyurethan aus einem Diisocyanat, Dimethylolpropansäure, Polyesterdiol und aliphatischem Diol.
◆ Polyurethan des Standes der Technik, DynamX® POLYMER der Firma National Starch, INCI-Name : Polyurethane 14 (and) AMP-Acrylates Copolymer, Polyurethane-14 besteht aus Isophorondiisocyanat, Propylenglykol, Dimethylpropansäure, Diol, Polyether.
▲ Erfindungsgemäßes Polyurethan gemäß Beispiel 4

Die Versuche zeigen deutlich, das mit dem erfindungsgemäß verwendeten Polyurethan der beste Lockenhalt erzielt wird.

### Anwendungstechnische Beispiele:

(Angegeben sind Gewichtsteile).

**"Pump-setting-Spray"**

| | A | B |
|---|---|---|
| Erfindungsgemäßes Polyurethan (bezogen auf Feststoff) | 2 | 10 |
| Ethanol | 55 | 30 |
| Parfüm | q.s. | q.s. |
| Wasser | ad 100 | ad 100 |

**Aerosol-Hair-Sprays**

| | C | D | E | F | G |
|---|---|---|---|---|---|
| Erfindungsgemäßes Polyurethan (bezogen auf Feststoff) | 5 | 10 | 2 | 5 | 8 |
| Octylacrylamid/acrylat/Butylaminoethyl methacrylat¹ (bezogen auf Feststoff) | | | | 1,8 | |
| Acrylat-Copolymer² | | | 2 | | |
| Aminomethylpropanol | | | q.s | q.s | |
| Glycerin | | 0,5 | | | |
| Panthenol | | | 0,5 | | 0,5 |
| PEG/PPG-18/18 Dimethicone | | | | 0,5 | |
| PEG-12 dimethicone | | 0,05 | | | |
| Propylene Glycol | | | | 0,5 | |
| Cyclomethicone | | | 1,0 | | 1,0 |
| Benzophenone-3 | | 0,1 | 0,1 | | 0,1 |
| Parfüm | q.s | q.s | q.s | q.s | q.s |
| Ethanol | 14,5 | 20 | 60 | 30 | 20 |
| Wasser | ad 100 | ad 100 | ad 100 | ad 100 | ad 100 |
| Propan/butan 3,5 Bar. (20°C) | | | 20 | 10 | |
| Dimethylether | 40 | 30 | | 30 | 20 |
| Fluorkohlenwasserstoff 152 A | | | | | 20 |

| | | | | | |
|---|---|---|---|---|---|
| ¹ Amphomer, National starch ² Luvimer P-100, BASF | | | | | |

**Haarschaum**

| | H | I |
|---|---|---|
| Erfindungsgemäßes Polyurethan (bezogen auf Feststoff) | 2 | 4 |
| Glycerin | 0,1 | |
| Panthenol | 0,05 | 0,5 |
| Polyquaternium-4 | 2 | |
| Cetyltrimethylammoniumchlorid | 0,2 | 0,5 |
| PEG-12 dimethicone | | 0,5 |
| Cyclomethicone | | 0,5 |
| Benzophenone- 3 | 0,1 | |
| Parfüm | q.s. | q.s. |
| Ethanol | 15 | 10 |
| Wasser | ad 100 | ad 100 |
| Konservierungsmittel | q.s. | q.s. |
| Dimethylether | 10 | 7 |
| Fluorkohlenwasserstoff 152 A | | 3 |

**Haargel/creme**

| | J | K | L |
|---|---|---|---|
| Erfindungsgemäßes Polyurethan (bezogen auf Feststoff) | 5 | 2 | 8 |
| Carbomer | 0,8 | | |
| Acrylsäure/VP Copolymer | | 0,5 | |
| Ammonium acryloyldimethyltaurat/VP Copolymer | | | 0,8 |
| Glycerin | 0,5 | | |
| Panthenol | | 0,5 | 0,5 |
| Propylenglycol | | | 0,2 |
| Cyclomethicone | | 0,2 | |
| Neutralisierungsmittel | q.s. | q.s. | q.s. |
| Parfüm | q.s. | q.s. | q.s. |
| Ethanol | 20 | | |
| Wasser | ad. 100 | ad. 100 | ad. 100 |
| Konservierungsmittel | q.s. | q.s. | q.s. |

## Patentansprüche

1. Haarfestiger-Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist.

2. Haarfestiger-Zusammensetzung nach Anspruch 1, worin die aminofunktionellen Verbindungen B) aus primären und/oder sekundären Aminen und/oder Diaminen ausgewählt werden.

3. Haarfestiger-Zusammensetzung nach einem der Ansprüche 1 oder 2, worin die aminofunktionelle Verbindungen B) mindestens ein Diamin umfassen.

4. Haarfestiger-Zusammensetzung nach einem der Ansprüche 1 bis 3, worin die aminofunktionellen Verbindungen B) wenigstens eine aminofunktionelle Verbindung B2) umfassen, die 2-(2-Amino-ethylamino)ethansulfonsäure und/oder deren Salze ist.

5. Haarfestiger-Zusammensetzung nach einem der Ansprüche 1 bis 4, worin die aminofunktionellen Verbindungen B) wenigstens eine aminofunktionelle Verbindung B1) umfassen, die keine ionische und/oder ionogene Gruppen aufweisen, bevorzugt ein Diamin, dass keine ionischen und/oder ionogenen Gruppen aufweist,

6. Haarfestiger-Zusammensetzung nach einem der Ansprüche 1 bis 5, worin die aminofunktionellen Verbindungen B) sowohl aminofunktionelle Verbindungen B2), die ionische und/oder ionogene Gruppen aufweisen, als auch aminofunktionellen Verbindungen B1), die keine ionischen und/oder ionogenen Gruppe aufweisen, umfassen.

7. Haarfestiger-Zusammensetzung nach einem der Ansprüche 1 bis 6, worin die Prepolymere A) erhältlich sind durch die Umsetzung von einem oder mehreren Polyolen, ausgewählt aus der Gruppe, die aus Polyether-Polyolen, Polycarbonatpolyolen, Polyether-Polycarbonat-Polyolen und/oder Polyesterpolyolen besteht, und einem oder mehreren Polyisocyanaten.

8. Haarfestiger-Zusammensetzung nach einem der Ansprüche 1 bis 7, worin das Polyurethan mindestens eine Sulfonsäure und/oder Sulfonatgruppe, bevorzugt eine Natriumsulfonatgruppe enthält.

9. Haarfestiger-Zusammensetzung nach einem der Ansprüche 1 bis 8, **dadurch gekennzeichnet, dass** sie einen Gehalt an flüchtigen organischen Verbindungen (VOC) von weniger als 80 Gew.-% bezogen auf die Haarfestiger-Zusammensetzung aufweist.

10. Verwendung einer kosmetischen Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist zur Gestaltung und/oder Festigung der Haare

11. Verwendung von Polyurethanen, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist, zur Herstellung von kosmetischen Zusammensetzungen zur Gestaltung oder Festigung der Haare.

12. Verfahren zur Gestaltung oder Festigung der Haare, welches das Auftragen einer kosmetischen Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A), wobei der Gehalt an ionischen und ionogenen Gruppen im Polyurethanprepolymer unter 15 Milliequivalenten pro 100 g Polyurethanprepolymer A) liegt, mit einer oder mehreren aminofunktionellen Verbindungen B), wobei die aminofunktionellen Verbindungen B) wenigstens eine aminofunktionelle Verbindung B2) umfassen, die Sulfonat- oder Sulfonsäuregruppen aufweist, auf das Haar, umfasst.

## Claims

1. Hair setting composition comprising at least one polyurethane obtainable by reacting one or more water-insoluble, non-water-dispersible, isocyanate-functional polyurethane prepolymers A), wherein the content of ionic and ionogenic groups in the polyurethane prepolymer is below 15 milliequivalents per 100g of polyurethane prepolymer A), with one or more amino-functional compounds B), wherein the amino-functional compounds B) include at least one amino-functional compound B2) which has sulfonate or sulfonic acid groups.

2. Hair setting composition according to Claim 1, wherein the amino-functional compounds B) are selected from primary and/or secondary amines and/or diamines.

3. Hair setting composition according to either of Claims 1 and 2, wherein the amino-functional compounds B) include at least one diamine.

4. Hair setting composition according to one of Claims 1 to 3, wherein the amino-functional compounds B) include at least one amino-functional compound B2) which is 2-(2-aminoethylamino)ethane sulfonic acid and/or salts thereof.

5. Hair setting composition according to one of Claims 1 to 4, wherein the amino-functional compounds B) include at least one amino-functional compound B1) that have no ionic and/or ionogenic groups, preferably a diamine which has no ionic and/or ionogenic groups.

6. Hair setting composition according to one of Claims 1 to 5, wherein the amino-functional compounds B) include both amino-functional compounds B2) which have ionic and/or ionogenic groups, and also amino-functional compounds B1) which have no ionic and/or ionogenic group.

7. Hair setting composition according to one of Claims 1 to 6, wherein the prepolymers A) are obtainable by reacting one or more polyols selected from the group which consists of polyether polyols, polycarbonate polyols, polyether-polycarbonate polyols and/or polyester polyols, and one or more polyisocyanates.

8. Hair setting composition according to one of Claims 1 to 7, wherein the polyurethane contains at least one sulfonic acid and/or sulfonate group, preferably a sodium sulfonate group.

9. Hair setting composition according to one of Claims 1 to 8, **characterized in that** it has a content of volatile organic compounds (VOC) of less than 80% by weight, based on the hair setting composition.

10. Use of a cosmetic composition comprising at least one polyurethane obtainable by reacting one or more water-insoluble, non-water-dispersible, isocyanate-functional polyurethane prepolymers A), wherein the content of ionic and ionogenic groups in the polyurethane prepolymer is below 15 milliequivalents per 100g of polyurethane prepolymer A), with one or more amino-functional compounds B), wherein the amino-functional compounds B) include at least one amino-functional compound B2) which has sulfonate or sulfonic acid groups, for shaping and/or setting the hair.

11. Use of polyurethanes obtainable by reacting one or more water-insoluble, non-water-dispersible, isocyanate-functional polyurethane prepolymers A), wherein the content of ionic and ionogenic groups in the polyurethane prepolymer is below 15 milliequivalents per 100g of polyurethane prepolymer A), with one or more amino-functional compounds B), wherein the amino-functional compounds B) include at least one amino-functional compound B2) which has sulfonate or sulfonic acid groups, for the preparation of cosmetic compositions for shaping or setting the hair.

12. Method of shaping or setting the hair, which involves applying a cosmetic composition, comprising at least one polyurethane obtainable by reacting one or more water-insoluble, non-water-dispersible, isocyanate-functional polyurethane prepolymers A), wherein the content of ionic and ionogenic groups in the polyurethane prepolymer is below 15 milliequivalents per 100g of polyurethane prepolymer A), with one or more amino-functional compounds B), wherein the amino-functional compounds B) include at least one amino-functional compound B2) which has sulfonate or sulfonic acid groups, to the hair.

## Revendications

1. Composition de fixateur capillaire, contenant au moins un polyuréthane, pouvant être obtenu par mise en réaction d'un ou de plusieurs prépolymères de polyuréthane à fonction isocyanate insolubles dans l'eau, non dispersibles dans l'eau A), la teneur en groupes ioniques et ionogènes dans le prépolymère de polyuréthane étant inférieure à 15 milliéquivalents pour 100 g de prépolymère de polyuréthane A), avec un ou plusieurs composés à fonction amino B), les composés à fonction amino B) comprenant au moins un composé à fonction amino B2) qui comprend des groupes sulfonate ou acide sulfonique.

2. Composition de fixateur capillaire selon la revendication 1, dans laquelle les composés à fonction amino B) sont choisis parmi les amines et/ou diamines primaires et/ou secondaires.

3. Composition de fixateur capillaire selon l'une quelconque des revendications 1 ou 2, dans laquelle les composés à fonction amino B) comprennent au moins une diamine.

4. Composition de fixateur capillaire selon l'une quelconque des revendications 1 à 3, dans laquelle les composés à fonction amino B) comprennent au moins un composé à fonction amino B2), qui est l'acide 2-(2-amino-éthylamino)éthane-sulfonique et/ou ses sels.

5. Composition de fixateur capillaire selon l'une quelconque des revendications 1 à 4, dans laquelle les composés à fonction amino B) comprennent au moins un composé à fonction amino B1) qui ne comprend pas de groupes ioniques et/ou ionogènes, de préférence une diamine qui ne comprend pas de groupes ioniques et/ou ionogènes.

6. Composition de fixateur capillaire selon l'une quelconque des revendications 1 à 5, dans laquelle les composés à fonction amino B) comprennent aussi bien des composés à fonction amino B2) qui comprennent des groupes ioniques et/ou ionogènes, que des composés à fonction amino B1) qui ne comprennent pas de groupes ioniques et/ou ionogènes.

7. Composition de fixateur capillaire selon l'une quelconque des revendications 1 à 6, dans laquelle les prépolymères A) peuvent être obtenus par la mise en réaction d'un ou de plusieurs polyols, choisis dans le groupe qui est constitué par les polyéther-polyols, les polycarbonate-polyols, les polyéther-polycarbonate-polyols et/ou les polyester-polyols, et d'un ou de plusieurs polyisocyanates.

8. Composition de fixateur capillaire selon l'une quelconque des revendications 1 à 7, dans laquelle le polyuréthane contient au moins un groupe acide sulfonique et/ou sulfonate, de préférence un groupe sulfonate de sodium.

9. Composition de fixateur capillaire selon l'une quelconque des revendications 1 à 8, **caractérisée en ce qu'**elle présente une teneur en composés organiques volatils (COV) de moins de 80 % en poids, par rapport à la composition de fixateur capillaire.

10. Utilisation d'une composition cosmétique, contenant au moins un polyuréthane, pouvant être obtenu par mise en réaction d'un ou de plusieurs prépolymères de polyuréthane à fonction isocyanate insolubles dans l'eau, non dispersibles dans l'eau A), la teneur en groupes ioniques et ionogènes dans le prépolymère de polyuréthane étant inférieure à 15 milliéquivalents pour 100 g de prépolymère de polyuréthane A), avec un ou plusieurs composés à fonction amino B), les composés à fonction amino B) comprenant au moins un composé à fonction amino B2) qui comprend des groupes sulfonate ou acide sulfonique, pour la mise en forme et/ou la fixation des cheveux.

11. Utilisation de polyuréthanes, pouvant être obtenus par mise en réaction d'un ou de plusieurs prépolymères de polyuréthane à fonction isocyanate insolubles dans l'eau, non dispersibles dans l'eau A), la teneur en groupes ioniques et ionogènes dans le prépolymère de polyuréthane étant inférieure à 15 milliéquivalents pour 100 g de prépolymère de polyuréthane A), avec un ou plusieurs composés à fonction amino B), les composés à fonction amino B) comprenant au moins un composé à fonction amino B2) qui comprend des groupes sulfonate ou acide sulfonique, pour la fabrication de compositions cosmétiques pour la mise en forme ou la fixation des cheveux.

12. Procédé de mise en forme ou de fixation des cheveux, qui comprend l'application d'une composition cosmétique, contenant au moins un polyuréthane, pouvant être obtenu par mise en réaction d'un ou de plusieurs prépolymères de polyuréthane à fonction isocyanate insolubles dans l'eau, non dispersibles dans l'eau A), la teneur en groupes ioniques et ionogènes dans le prépolymère de polyuréthane étant inférieure à 15 milliéquivalents pour 100 g de prépolymère de polyuréthane A), avec un ou plusieurs composés à fonction amino B), les composés à fonction amino B) comprenant au moins un composé à fonction amino B2) qui comprend des groupes sulfonate ou acide sulfonique, sur les cheveux.
